# EUROPÄISCHE PATENTSCHRIFT

(11) **EP 1 322 589 B1**
(45) Veröffentlichungstag und Bekanntmachung des Hinweises auf die Patenterteilung: **28.06.2006**
(21) Anmeldenummer: 01976202.0
(22) Anmeldetag: 07.09.2001
(51) Int. Cl.: C07C 67/54, C07C 69/14

(54) **KOPPELPRODUKTION ZWEIER ESTER**
COUPLED PRODUCTION OF TWO ESTERS
PRODUCTION COUPLEE DE DEUX ESTERS

(30) Priorität: 16.09.2000 DE 10045894
(43) Veröffentlichungstag der Anmeldung: 02.07.2003
(73) Patentinhaber: Celanese Chemicals Europe GmbH, 60439 Frankfurt am Main (DE)
(72) Erfinder: SCHULZ, Eckhard, 61350 Bad Homburg (DE); BAUER, Helmut, 65931 Frankfurt (DE); MERSCHER, Klaus, Dieter, 64572 Büttelborn (DE)
(86) Internationale Anmeldenummer: PCT/EP2001/010349
(87) Internationale Veröffentlichungsnummer: WO 2002/022547

(56) Entgegenhaltungen:
- WO-A-98/42652
- GB-A- 1 394 651

## Beschreibung

Die Erfindung betrifft die Koppelproduktion zweier Ester durch Umsetzung eines Gemisches aliphatischer Alkohole mit einer aliphatischen Carbonsäure und anschließender destillativer Aufarbeitung des Gemisches in die reinen Esterverbindungen.

Ester aliphatischer Alkohole und Carbonsäuren besitzen als Lösungsmittel eine große wirtschaftliche Bedeutung. So finden beispielsweise Ethylacetat oder n-Butylacetat als Lösungsmittel in der Farben- und Lackindustrie eine breite Anwendung. Auch bei der Herstellung von Cellophan, Celluloid, Collodiumwolle und Kunstharzen werden Ester aliphatischer Alkohole und aliphatischer Carbonsäuren eingesetzt. Acrylsäureester, wie zum Beispiel Butylacrylat oder 2-Ethylhexylacrylat finden bevorzugt Anwendung auf dem Anstrich- und Klebesektor sowie in der Papier-, Textil- und Lederveredelung. Ferner sind Acrylsäureester wichtige Monomere bei der Herstellung von Polyacrylaten.

Die unmittelbare Veresterung von Alkoholen mit Carbonsäuren gehört zu den Grundoperationen der organischen Chemie. Um die Reaktion in Richtung des gewünschten Esters zu lenken, ist es zweckmäßig, das Reaktionswasser kontinuierlich zu entfernen. Geeignete Maßnahmen sind zum Beispiel die Azeotropdestillation, das Erhitzen des Reaktionsgemisches unter Durchleiten eines Inertgases, die Durchführung der Umsetzung unter Vakuum oder die Anwesenheit eines Trockenmittels. Die Azeotropdestillation kann in Gegenwart eines mit Wasser nicht mischbaren zugesetzten Schleppmittels erfolgen. Es ist aber auch möglich, daß im Überschuß vorhandene Säure oder Alkohol als Schleppmittel dient. Dabei wählt man zweckmäßigerweise die Komponente im Überschuß, die den geringeren Siedepunkt aufweist.

Die Reaktion kann in Abwesenheit von Katalysatoren erfolgen, erfordert dann aber höhere Temperaturen und längere Reaktionszeiten. Beide Reaktionsbedingungen können durch den Einsatz saurer Katalysatoren gemildert werden. Neben Schwefelsäure sind organische Säuren wie Methansulfonsäure, p-Toluolsulfonsäure sowie Kationenaustauscher vom Polystyrolsulfonsäure-Typ die bevorzugten Katalysatoren.

Wichtiges Qualitätskriterium für die gewünschten Esterverbindungen sind Alkoholrestgehalte, die für viele Anwendungsgebiete bestimmte Grenzwerte nicht übersteigen dürfen. Um diese Qualitätsanforderung zu erfüllen, ist häufig eine gesonderte Reindestillation der Esterverbindungen erforderlich.

Nach GB-1,394,651 erhält man Butylacetat, wenn man ein Gemisch aus Ethanol und Butanol mit Essigsäure verestert und anschließend das Rohgemisch destillativ aufarbeitet. Nach dem aus GB-1,394,651 bekannten Verfahren liefert die Veresterung eines Gemisches, bestehend aus 66,7 % Gew.-% Ethanol und 33,3 Gew.-% Butanol ein am Kopf der Veresterungskolonne anfallendes Rohgemisch, das in einer ersten Destillationskolonne von Resten des Reaktionswassers und überschüssigem Alkohol befreit und über den Sumpfabzug auf eine zweite Destillationskolonne gegeben wird, in der als Kopfprodukt reines Ethylacetat und als Sumpfprodukt Butylacetat mit einer Reinheit von 98,2 % anfällt.

Gegenüber der getrennten Herstellung der Esterverbindungen zeichnet sich die aus GB-1,394,651 bekannte Koppelproduktion durch ein geringeres Reaktionsvolumen und Destillationsvolumen aus. Die Koppelproduktion führt daher zu Einsparungen bei den Investitionskosten und beim Dampfverbrauch.

Trotz der geschilderten Vorteile führt das nach dem Vorschlag von GB-1,394,651 durchzuführende Destillationsverfahren zu einem Butylacetat mit erheblichen Restgehalten an n-Butanol, denn die die Veresterungskolonne über Kopf verlassenden Restmengen an n-Butanol gelangen komplett in die Butylacetatreinfraktion. Für viele Anwendungsgebiete ist es jedoch erforderlich, ein Butylacetat mit einem möglichst geringen Restgehalt an n-Butanol bereitzustellen.

Ein weiteres Verfahren zur Koppelproduktion von Ethyl- und Butylacetat ist aus WO98/42652 bekannt. Dabei wird das rohe Veresterungsgemisch nach Abtrennung der flüchtigen Anteile auf eine Trennkolonne gegeben und in Ethylacetat als Kopfprodukt und Butylacetat als Sumpfprodukt aufgetrennt. Dabei wird der Trennkolonne ein flüssiger Seitenstrom entnommen, der Verunreinigungen, beispielsweise Butanol oder Crotonaldehyd, enthält und der verworfen wird. Nach der Lehre von WO98/42652 lassen sich Verunreinigungen, die die Carboxylgruppe enthalten, durch Behandlung mit einem polymeren Harz entfernen, wobei die Ausgangsverbindungen oder die fertigen Esterprodukte mit einem polymeren Harz behandelt werden können, das z.B. mit Bisulfit-Ionen beladen wird, um Aldehydverunreinigungen zu fixieren.

Es bestand daher die Aufgabe ein Verfahren zur Koppelproduktion zweier Ester bereitzustellen, bei dem beide Esterverbindungen mit ausgezeichneter Reinheit und geringen Restgehalten an Ausgangsalkoholen erhalten werden. Gelöst wird diese Aufgabe durch ein Verfahren zur Koppelproduktion von Ethyl- und Butylacetat oder Ethyl- und i-Propylacetat durch Umsetzung eines Gemisches von Ethanol und Butanol oder von Ethanol und i-Propanol mit Essigsäure und destillativer Aufarbeitung des Rohestergemisches. Es ist dadurch gekennzeichnet, daß das den Veresterungsreaktor verlassende Rohestergemisch nach Abtrennung des Reaktionswassers auf eine erste Destillationskolonne gegeben wird, in der unterhalb der Aufgabestelle des Rohesters ein die höher siedende Alkoholkomponente enthaltender Seitenstrom abgezogen und in den Veresterungsreaktor zurückgeführt wird, in der die höher siedende Esterverbindung über den Sumpf abgezogen wird und in der der Kopfabzug nach Abtrennung von Wasser auf eine zweite Destillationskolonne gegeben wird, in der über den Sumpf die niedrig siedende Esterverbindung abgezogen wird und in der der Kopfabzug nach Abtrennung von Wasser in den Veresterungsreaktor zurückgeführt wird.

Überraschenderweise enthält die gewünschte niedrig siedende bzw. höher siedende Esterverbindung, die nach dem erfindungsgemäßen Verfahren über den Sumpf der ersten bzw. zweiten Destillationskolonne abgezogen wird, nur sehr geringe Restmengen an den Ausgangsalkoholen. Sie fallen in einer für die meisten Anwendungen ausreichenden Qualität an und können ohne weitere Reinigungsschritte eingesetzt werden.

Das erfindungsgemäße Verfahren gestattet die Koppelproduktion von Esterverbindungen, die aus Essigsäure und den aliphatischen Alkoholen Ethanol, iso-Propanol und n-Butanol aufgebaut sind.

Die als Ausgangsstoffe verwendeten Alkohole sind industriell produzierte Chemikalien.

Die Essigsäure und die beiden Komponenten des Alkoholgemisches sind bezüglich ihrer Siedepunkte bzw. bezüglich ihrer sich in Gegenwart des auszuschleusenden Wassers bildender Azeotrope so auszuwählen, daß die Essigsäure den höchsten Siedepunkt unter den drei Ausgarigsverbindungen aufweist.

Eine solche Wahl der Ausgangsverbindungen stellt sicher, daß die Essigsäure komplett im Veresterungsreaktor zurückgehalten wird.

Das zur Koppelproduktion zweier Carbonsäureester eingesetzte Gemisch zweier Alkohole kann in seiner Zusammensetzung über weite Bereiche variieren. Im allgemeinen liegt die Zusammensetzung des Alkoholgemisches im Bereich von 90 :10 Gew.-% bis zu 10 : 90 Gew.-%, vorzugsweise im Bereich von 80 : 20 Gew.-% bis zu 20 : 80 Gew.-% und insbesondere von 70 : 30 Gew.-% bis zu 30 : 70 Gew.-% an den jeweiligen Alkoholen, bezogen auf die Gesamtmasse des einzusetzenden Alkoholgemisches. Vorzugsweise arbeitet man mit Alkoholgemischen, bei denen sich die Azeotrope günstig in Bezug auf ihren Wasserbedarf ergänzen.

Im Falle der Koppelproduktion von Ethyl- und Butylacetat lassen sich bei einer Alkoholeinsatzmischung mit einem Ethandlanteil von 70 Gew.-% und einem Butanolanteil von 30 Gew.-%, bezogen auf die Gesamtmasse des einzusetzenden Alkoholgemisches, besonders günstige Azeotrope einstellen. Bei der Koppelproduktion von Ethyl- und Isopropylacetat empfiehlt es sich, ein Ausgangsgemisch mit einem Ethanolgehalt von 80 Gew.-% und einem Isopropanolgehalt von 20 Gew.-%, bezogen auf die Gesamtmasse des einzusetzenden Alkoholgemisches, zu verwenden, um eine besonders vorteilhafte Azeotropbildung auszunutzen.

Die in den Veresterungsreaktor eingespeiste Menge an Alkoholen entspricht in ihrer Summe im allgemeinen den eingespeisten Äquivalenten an Essigsäure, da nicht umgesetzter Alkohol im allgemeinen aus dem Reaktionswasser ausgestrippt und in die Reaktion zurückgeführt wird. Daher reicht es bei der kontinuierlich durchgeführten Veresterungsreaktion aus, die Menge an Alkoholen entsprechend den vorliegenden Carbonsäureäquivalenten einzusetzen. Ebenfalls fungiert der Alkohol als Azeotropbildner und dient so als Schleppmittel zur Entfernung des Reaktionswassers.

Weitere Verfahrensvarianten bestehen darin, das Reaktionswasser unter Durchleiten eines Inertgases oder unter Anlegen eines Unterdrucks zu entfernen.

Die Veresterung von Carbonsäuren mit Alkoholen ist ein aus dem Stand der Technik bekanntes Verfahren. Die Veresterung kann in Abwesenheit eines Katalysators erfolgen, mit dem Vorteil, daß man den Zusatz eines Fremdstoffes vermeidet. In diesem Falle wirkt die Carbonsäure gleichzeitig als Reaktionskomponente für den Alkohol und als Katalysator. Allerdings muß man dann im allgemeinen höhere Reaktionstemperaturen einhalten, um die Umsetzung mit einer ausreichend hohen Geschwindigkeit ablaufen zu lassen. Ferner kann eine übermäßige thermische Belastung zu einer Schädigung der Ester und einer Farbvertiefung führen.

Im allgemeinen setzt man jedoch übliche Veresterungskatalysatoren, wie zum Beispiel Schwefelsäure, Phosphorsäure, p-Toluoisulfonsäure oder Methansulfonsäure zu. Um Korrosionserscheinungen zu vermeiden oder zu behindern verwendet man bevorzugt Methan- oder p-Toluolsulfonsäure. Die Konzentration des Veresterungskatalysators beträgt im allgemeinen 0,5 bis 3 Gew.-%, bezogen auf die gesamte Reaktionsmischung.

Die Veresterungsreaktion wird im allgemeinen bei Normaldruck oder bei Drücken von 0,001 bis 0,3 MPa und bei Temperaturen im Bereich von 60 bis 180°C durchgeführt. Die jeweils einzustellenden Reaktionsbedingungen richten sich nach den verwendeten Einsatzstoffen und der gewählten Verfahrensvariante, ob zum Beispiel die Reaktion in Gegenwart eines Katalysators durchgeführt wird oder ob zur Entfernung des Reaktionswassers ein Unterdruck angelegt wird.

Vorzugsweise führt man die Veresterungsreaktion kontinuierlich in einer Veresterungskolonne durch, in der der Kolonnensumpf, auch als Blase bezeichnet, als Reaktionsgefäß und der aufgesetzte Kolonnenturm als Destillationskolonne zur Rektifikation und Entfernung der bereits gebildeten Esterverbindungen, des nicht reagierten Alkohols und des Reaktionswassers dient, wobei gleichzeitig die der Veresterungsreaktion folgende Rektifikation die Carbonsäure komplett in der Blase zurückhält. Der Kolonnendruck, die Kolonnentemperatur und das am Kolonnenkopf einzustellende Rücklaufverhältnis richten sich nach den jeweils einzusetzenden Ausgangsverbindungen. Üblicherweise wird bei Normaldruck gearbeitet. Kolonnentemperatur, Kopftemperatur und Rücklaufverhältnis werden so eingestellt, daß über den Kolonnenkopf keine Carbonsäure in den abdestillierten Ester gelangt gleichzeitig aber genügend Reaktionswasser ausgetragen wird.

Das den Kolonnenkopf verlassende Gemisch wird kondensiert und gelangt nach Abtrennung des als Rücklauf in die Veresterungskolonne zurückgeführten Anteils in den Phasentrenner, wobei sich Reaktionswasser und organische Phase voneinander scheiden. Je nach Wahl der Ausgangsverbindungen kann der Rücklauf durch Zugabe von organischer oder wäßriger Phase zur Azeotropbildung ergänzt werden. Das anfallende organische Rohestergemisch wird anschließend gemäß dem erfindungsgemäßen Verfahren destillativ gereinigt.

Das in einem Kessel nach diskontinuierlicher oder bevorzugt in einer Veresterungskolonne nach kontinuierlicher Verfahrensvariante erhaltene Rohestergemisch wird anschließend auf eine erste Destillationskolonne gegeben, vorzugsweise auf den mittleren Bereich der Kolonne, besonders bevorzugt zwischen dem 15. und 25. Boden, gerechnet vom Kolonnenfuß. Unterhalb der Zugabestelle des Rohesters, vorzugsweise im Abtriebsteil und besonders bevorzugt zwischen dem 2. und 10. Boden, vom Kolonnenfuß gerechnet, wird ein Stoffstrom, in dem die Alkoholkomponente mit dem höheren Siedepunkt angereichert ist, abgezogen und in den Veresterungsreaktor zurückgeführt. Über den Sumpf der ersten Destillationskolonne wird die reine höher siedende Esterverbindung, zum Beispiel Butylacetat, abgezogen, während die flüchtigen Bestandteile, bestehend überwiegend aus der leichter siedenden Esterverbindung, verunreinigt mit geringen Anteilen an dem niedrig siedenden Alkohol und Restwassermengen, über den Kopf der ersten Destillationskolonne entfernt werden. Kolonnentemperatur, Kolonnendruck und Rücklaufverhältnis in der ersten Destillationskolonne richten sich nach dem jeweils aufzutrennenden Estergemisch.

Der den Kolonnenkopf der ersten Destillationskolonne verlassende niedrig siedende Rohester, z.B. rohes Ethylacetat, wird kondensiert und nach Abtrennung des für den Rücklauf in die erste Destillationskolonne benötigten Anteils auf einen Phasentrenner gegeben, wobei Restwassermengen abgeschieden werden. Das organische Rohprodukt wird anschließend in einer zweiten Destillationskolonne von weiteren Restwassermengen sowie von den Anteilen an dem niedrig siedenden Alkohol gereinigt. Der Zufluß auf die zweite Destillationskolonne erfolgt vorzugsweise auf den mittleren Bereich der zweiten Destillationskolonne, besonders auf den 10. bis 20. Boden, gerechnet vom Kolonnenfuß. Über den Sumpf wird die reine niedrig siedende Esterverbindung, z.B. reines Ethylacetat, abgezogen, während das Kopfprodukt nach Kondensation und Abtrennung des für den Rücklauf in die zweite Destillationskolonne benötigten Anteils zunächst auf einen Phasentrenner gegeben wird. Die organische Phase wird in den Veresterungsreaktor zurückgeführt. Die mit Alkohol- und Esterverbindungen gesättigte Wasserphase wird mit den Wasserphasen, die man nach der Phasentrennung der kondensierten Kopfprodukte aus der Veresterungs- und aus der ersten Destillationskolonne erhält, vereinigt und in einer nachgeschalteten Wasserstrippkolonne von den organischen Bestandteilen befreit. Die dabei zurückgewonnenen Ausgangsalkohole werden in den Veresterungsreaktor zurückgeführt.

Die Koppelproduktion von zwei Ersterverbindungen stellt ein besonders wirtschaftliches Verfahren dar, da durch die Einsparungen von Kolonnen im Veresterungsteü und im Aufarbeitungsteil die Investionskosten gesenkt werden können. Ein reduzierter Betrieb von Destillationskolonnen vermindert ebenfalls den Dampfverbrauch, was zu einer zusätzlichen Energieeinsparung führt. Weiterhin lassen sich durch nur eine einzige Steuerung zwei Destillationsprozesse regeln. Schließlich führt die Nutzung der Azeotrope zu einer effizienteren Wasserauschleusung, so daß weniger Wasser im Kreislauf zu fahren bzw. zu verdampfen ist.

Überraschenderweise lassen sich über die Sumpfabzüge aus den Destillationskolonnen die beiden Esterverbindungen in ausgezeichneter Reinheit gewinnen, die ohne weitere Reinigungsschritte für die meisten Anwendungen eingesetzt werden können. Die Ausschleusung der Reinprodukte über den Sumpf ist besonders deshalb vorteilhaft, weil das Hauptprodukt nicht nochmals komplett verdampft werden muß und das Rückverhältnis sowie der Dampfeinsatz in den Destillationskolonnen über weite Bereiche variiert werden können, ohne daß die Qualität der über den Sumpf abgezogenen Esterverbindungen beeinträchtigt wird.

Selbst wenn die Zusammensetzung des Alkoholgemisches in weiten Grenzen variiert, die sich im Bereich von 90 : 10 Gew.-% bis 10 : 90 Gew.-% an den jeweiligen Alkoholen, bezogen auf die Gesamtmasse des einzusetzenden Alkohlgemisches, erstrecken kann, lassen sich nach dem erfindungsgemäßen Verfahren die gewünschten Esterverbindungen in ausgezeichneter Reinheit gewinnen.

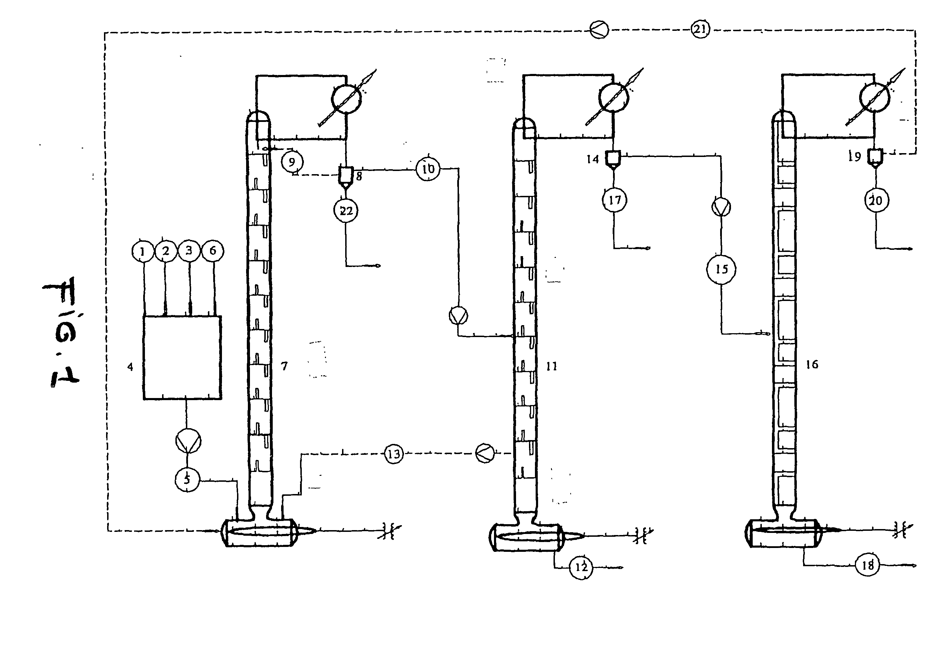

Im folgenden wird eine beispielhafte Ausgestaltung der Erfindung anhand des in der Figur dargestellten Verfahrensfließbildes näher beschrieben. Eine Beschränkung der Erfindung in irgendeiner Weise ist dadurch nicht beabsichtigt.

Der bekannte Teil des Verfahrens wurde weiter oben bereits beschrieben, weshalb hier nur die Bezugsziffern genannt seien: 1 Essigsäure, 2 und 3 jeweiliger aliphatischer Alkohol, 4 Vormischer, Stoffstrom 5, dem Sumpf der Veresterungskolonne 7 zugeführtes Einsatzgemisch, 6 Katalysator, 7 Veresterungskolonne mit Katalysator im Sumpf, 8 Phasentrenner, 9 Azeotropergänzung, Stoffstrom 10 aus dem Phasentrenner 8 abgezogenes organisches Rohgemisch, Stoffstrom 22 aus dem Phasentrenner 8 abgezogene Wasserphase.

Erfindungsgemäß wird der Stoffstrom 10 auf den mittleren Bereich der ersten Destillationskolonne 11 gegeben. Über den Sumpf der Destillationskolonne 11 wird die reine Esterverbindung über den Stoffstrom 12 abgezogen, die den höheren Siedepunkt der beiden nach der Koppelproduktion hergestellten Esterverbindungen aufweist. Unterhalb der Zugabestelle des Rohesters wird in der Destillationskolonne 11 im Abtriebsteil der Stoffstrom 13 mit einem Anteil an dem höher siedenden Alkohol über einen Seitenabzug abgeführt und in den Sumpf der Veresterungskolonne 7 zurückgeführt. Der Kopfabzug wird nach Kondensation und Abtrennung des für den Rücklauf in die Destillationskolonne 11 benötigten Anteils auf den Phasentrenner 14 gegeben und in eine organische und wäßrige Phase geschieden. Die organische Phase, Stoffstrom 15, wird auf den mittleren Bereich der zweiten Destillationskolonne 16 gegeben, während die wäßrige Phase Stoffstrom 17 ausgeschleust wird. Über den Sumpfabzug Stoffstrom 18 der zweiten Destillationskolonne wird die reine niedrig siedende Esterverbindung erhalten während die flüchtigen Bestandteile nach Kondensation und Abtrennung des für das Rücklaufverhältnis erforderlichen Anteils auf den Phasentrenner 19 gegeben werden. Über den Phasentrenner 19 wird das Restwasser Stoffstrom 20 ausgetragen, während die abgetrennte organische Phase als Stoffstrom 21 in den Sumpf der Veresterungskolonne 7 wieder zurückgeführt wird.

Die an den Phasentrennern 8, 14 und 19 anfallenden Wasserströme 22, 17 und 20 können nach Vereinigung in bekannter Weise mittels einer Wasserstrippkolonne von den Restgehalten an Alkoholen und Esterverbindungen gereinigt werden. Die zurückgewonnenen Alkohole werden in den Veresterungsprozeß zurückgefahren.

Das folgende Beispiel sollen das erfindungsgemäße Verfahren beschreiben, ohne es jedoch einzuschränken.

### Koppelproduktion von Ethyl- und Butylacetat

In dem Vormischer 4 werden Ethanol 1, Butanol 2 und Essigsäure 3 abgemischt, so daß man ein Gemisch folgender prozentualer Zusammensetzung erhält:

| | |
|---|---|
| Ethanol | 24,6 Gew.-% |
| Butanol | 24,6 Gew.-% |
| Essigsäure | 50,8 Gew.-% |

das als Stoffstrom 5 in den Sumpf der Veresterungskolonne eingetragen wird. Der Sumpf der Veresterungskolonne enthält 2,0 Gew.-%, bezogen auf die gesamte Reaktionsmischung, Methansulfonsäure als Katalysator 6. Die Veresterungskolonne 7, ausgestattet mit 45 Böden, wird bei Normaldruck und einer Kopftemperatur von 83°C betrieben. Das Kopfprodukt wird kondensiert, abgekühlt und nach Abtrennung des als Rücklauf in die Veresterungskolonne 7 zurückgeführten Anteils auf den Phasentrenner 8 geleitet. Von der organischen Phase werden soviel Produkt 9 auf den Kopf der Veresterungskolonne zurückgeführt, daß sich ein Rücklaufverhältnis von 2 zu 3 einstellt, während der Rest als Stoffstrom 10 mit folgender Zusammensetzung

| | |
|---|---|
| Ethanol | 1,0 Gew.-% |
| Butanol | 0,6 Gew.-% |
| Essigsäure | 0,0 Gew.-% |
| Ethylacetat | 53,0 Gew.-% |
| Butylacetat | 43,1 Gew.-% |
| Wasser | 2,3 Gew.-% |

auf den 20. Boden (vom Kolonnenfuß gerechnet) der ersten Destillationskolonne 11 gegeben werden. Man betreibt die Destillationskolonne 11 bei Normaldruck und bei einer Kopftemperatur von 74°C. Von dem Kopfabzug der Destillationskolonne 11 wird nach Kondensation soviel Produkt auf den Kopf zurückgeführt, daß ein Rücklaufverhältnis von 1 zu 2 sichergestellt wird. Die restliche, am Kopf der Destillationskolonne 11 angefallene Produktmenge wird in dem Phasentrenner 14 in die organische Phase 15 und die wäßrige Phase 17 geschieden. Der aus dem Phasentrenner 14 abgeführte Stoffstrom 15 hat folgende Zusammensetzung

| | |
|---|---|
| Ethanol | 1,8 Gew.-% |
| Butanol | 0,0 Gew.-% |
| Essigsäure | 0,0 Gew.-% |
| Ethylacetat | 94,1 Gew.-% |
| Butylacetat | 0,0 Gew.-% |
| Wasser | 4,1 Gew.-%. |

Von dem 5. Boden, vom Kolonnenfuß gerechnet, der Destillationskolonne 11 entnimmt man einen Seitenstrom 13 folgender Zusammensetzung

| | |
|---|---|
| Ethanol | 0,0 Gew.-% |
| Butanol | 2,8 Gew.-% |
| Essigsäure | 0,0 Gew.-% |
| Ethylacetat | 0,8 Gew.-% |
| Butylacetat | 96,3 Gew.-% |
| Wasser | 0,1 Gew.-% |

und führt ihn in den Sumpf der Veresterungskolonne 7 zurück.

Über den Sumpf der Destillationskolonne 11 wird reines Butylacetat (Stoffstrom 12) folgender Zusammensetzung erhalten

| | |
|---|---|
| Ethanol | 0,0 Gew.-% |
| Butanol | 0,2 Gew.-% |
| Essigsäure | 0,0 Gew.-% |
| Ethylacetat | 0,0 Gew.-% |
| Butylacetat | 99,8 Gew.-% |
| Wasser | <0,1 Gew.-%. |

Der Stoffstrom 15 wird auf den mittleren Bereich (17. Boden, gerechnet vom Kolonnenfuß) der Destillationskolonne 16 gegeben, die bei Normaldruck und einer Kopftemperatur von 69°C betrieben wird. Über den Sumpf der Kolonne 16 wird reines Ethylacetat (Stoffstrom 18) mit folgender Zusammensetzung

| | |
|---|---|
| Ethanol | 0,1 Gew.-% |
| Butanol | 0,0 Gew.-% |
| Essigsäure | 0,0 Gew.-% |
| Ethylacetat | 99,9 Gew.-% |
| Butylacetat | 0,0 Gew.-% |
| Wasser | <0,1 Gew.-% |

gewonnen.

Von dem Kopfabzug der Destillationskolonne 16 wird nach Kondensation soviel Produkt auf den Kopf zurückgeführt, daß sich ein Rücklaufverhältnis von 3 zu 1 einstellt. Der restliche Anteil des Kopfprodukts wird auf den Phasentrenner 19 geleitet. Die abgetrennte organischen Phase wird als Stoffstrom 21 mit folgender Zusammensetzung

| | |
|---|---|
| Ethanol | 3,3 Gew.-% |
| Butanol | 0,0 Gew.-% |
| Essigsäure | 0,0 Gew.-% |
| Ethylacetat | 91,8 Gew.-% |
| Butylacetat | 0,0 Gew.-% |
| Wasser | 4,9 Gew.-% |

in den Sumpf der Veresterungskolonne 7 zurückgeführt.

Die an den Phasentrennern 8, 14 und 19 anfallenden Wasserströme 22, 17 und 20 haben folgende Zusammensetzung

### Stoffstrom 22

| | |
|---|---|
| Ethanol | 2,7 Gew.-% |
| Butanol | 0,2 Gew.-% |
| Essigsäure | 0,0 Gew.-% |
| Ethylacetat | 3,4 Gew.-% |
| Butylacetat | 0,3 Gew.-% |
| Wasser | 93,4 Gew.-% |

### Stoffstrom 17, 20

| | |
|---|---|
| Ethanol | 7,3 Gew.-% |
| Butanol | 0,0 Gew.-% |
| Essigsäure | 0,0 Gew.-% |
| Ethylacetat | 7,8 Gew.-% |
| Butylacetat | 0,0 Gew.-% |
| Wasser | 84,9 Gew.-% |

Wie die erfindungsgemäßen Beispiele zeigen, lassen sich bei der Koppelproduktion von zwei Esterverbindungen die gewünschten Esterverbindungen 12 und 18 über den Sumpf der beiden Destillationskolonnen 11 und 16 in einer solchen Reinheit gewinnen, daß sie ohne weitere Reinigungsschritte in den meisten Anwendungsgebieten eingesetzt werden können.

## Patentansprüche

1. Verfahren zur Koppelproduktion von Ethyl- und Butylacetat oder Ethyl- und i-Propylacetat durch Umsetzung eines Gemisches von Ethanol und Butanol oder von Ethanol und i-Propanol mit Essigsäure und destillativer Aufarbeitung des Rohestergemisches, **dadurch gekennzeichnet, daß** das den Veresterungsreaktor verlassende Rohestergemisch nach Abtrennung des Reaktionswassers auf eine erste Destillationskolonne gegeben wird, in der unterhalb der Aufgabestelle des Rohesters ein die höher siedende Alkoholkomponente enthaltender Seitenstrom abgezogen und in den Veresterungsreaktor zurückgeführt wird, in der die höher siedende Ester-verbindung über den Sumpf abgezogen wird und in der der Kopfabzug nach Abtrennung von Wasser auf eine zweite Destillationskolonne gegeben wird, in der über den Sumpf die niedrig siedende Esterverbindung abgezogen wird und in der der Kopfabzug nach Abtrennung von Wasser in den Veresterungsreaktor zurückgeführt wird.

2. Verfahren nach Anspruch 1, **dadurch gekennzeichnet, daß** man der ersten Destillationskolonne unterhalb der Aufgabestelle des Rohesters im Abtriebsteil, vorzugsweise am 2. bis 10. Boden den Seitenstrom entnimmt und in den Veresterungsreaktor zurückführt.

3. Verfahren nach Anspruch 1 oder 2 **dadurch gekennzeichnet, daß** man das Rohestergemisch auf den mittleren Bereich der ersten Destillationskolonne, vorzugsweise auf den 15. bis 25. Boden der ersten Destillationskolonne gibt.

4. Verfahren nach einem der Ansprüche 1 bis 3, **dadurch gekennzeichnet, daß** man den Kopfabzug aus der ersten Destillationskolonne auf den mittleren Bereich der zweiten Destillationskolonne, vorzugsweise auf den 10. bis 20. Boden der zweiten Destillationskolonne gibt.

5. Verfahren nach einem der Ansprüche 1 bis 4, **dadurch gekennzeichnet daß** der Anteil der jeweiligen Alkoholkomponente im Bereich von 10-90 Gew.-%, vorzugsweise 20-80 Gew.-% und besonders bevorzugt 30-70 Gew.-%, bezogen auf das Alkoholgemisch, liegt.

6. Verfahren nach einem der Ansprüche 1 bis 5, **dadurch gekennzeichnet, daß** die Veresterungsreaktion in Gegenwart eines Veresterungskatalysators mit einer Konzentration von 0,5 bis 3 Gew.-%, bezogen auf die gesamte Reaktionsmischung, durchgeführt wird.

7. Verfahren nach einem der Ansprüche 1 bis 6, **dadurch gekennzeichnet, daß** der Veresterungsreaktor eine Veresterungskolonne mit Kolonnensumpf und aufgesetztem Kolonnenturm ist.

## Claims

1. A process for the coupled production of ethyl acetate and butyl acetate or ethyl acetate and i-propyl acetate by reacting a mixture of ethanol and butanol or of ethanol and i-propanol with acetic acid and working up the crude ester mixture by distillation, wherein after the water of reaction has been separated off, the crude ester mixture leaving the esterification reactor is fed to a first distillation column, in which a side stream containing the higher-boiling alcohol component is taken off below the feed point of the crude ester and is recycled to the esterification reactor, in which the higher-boiling ester compound is taken off via the bottom and in which, after water has been separated off, the top take-off is fed to a second distillation column, in which the low-boiling ester compound is taken off via the bottom and in which, after water has been separated off, the top take-off is recycled to the esterification reactor.

2. The process as claimed in claim 1, wherein the side stream is removed from the first distillation column, below the feed point of the crude ester, in the stripping section, preferably at the 2nd to 10th tray, and is recycled to the esterification reactor.

3. The process as claimed in claim 1 or 2, wherein the crude ester mixture is fed to the middle region of the first distillation column, preferably to the 15th to 25th tray of the first distillation column.

4. The process as claimed in any of claims 1 to 3, wherein the top take-off from the first distillation column is fed to the middle region of the second distillation column, preferably to the 10th to 20th tray of the second distillation column.

5. The process as claimed in any of claims 1 to 4, wherein the amount of the respective alcohol component is in the range from 10-90% by weight, preferably 20-80% by weight and particularly preferably 30-70% by weight, based on the alcohol mixture.

6. The process as claimed in any of claims 1 to 5, wherein the esterification reaction is carried out in the presence of an esterification catalyst having a concentration of from 0.5 to 3% by weight, based on the total reaction mixture.

7. The process as claimed in any of claims 1 to 6, wherein the esterification reactor is an esterification column having a column bottom and attached column tower.

## Revendications

1. Procédé de production couplée d'acétate d'éthyle et de butyle ou d'acétate d'éthyle et de i-propyle par réaction d'un mélange d'éthanol et de butanol ou d'éthanol et de i-propanol avec de l'acide acétique et traitement par distillation du mélange brut d'esters, **caractérisé en ce que** le mélange brut d'esters quittant le réacteur d'estérification est, après séparation de l'eau réactionnelle, introduit dans une première colonne de distillation dans laquelle, en dessous de l'emplacement d'alimentation de l'ester brut, un courant latéral contenant le composant alcoolique à point d'ébullition élevé est soutiré et recyclé dans le réacteur d'estérification, dans laquelle le composé ester à point d'ébullition élevé est soutiré par le fond et dans laquelle l'effluent de tête est, après séparation de l'eau, introduit dans une deuxième colonne de distillation, dans laquelle le composé ester à bas point d'ébullition est soutiré par le fond et dans laquelle l'effluent de tête est, après séparation de l'eau, recyclé dans le réacteur d'estérification.

2. Procédé suivant la revendication 1, **caractérisé en ce que**, de la première colonne de distillation, on prélève le courant latéral en dessous de l'emplacement d'alimentation de l'ester brut dans la partie de rectification, de préférence au deuxième jusqu'au dixième plateau, et on le recycle dans le réacteur d'estérification.

3. Procédé suivant la revendication 1 ou 2,
**caractérisé en ce qu'**on introduit le mélange brut d'esters dans la zone centrale de la première colonne de distillation, de préférence au quinzième jusqu'au vingt-cinquième plateau de la première colonne de distillation.

4. Procédé suivant l'une des revendications 1 à 3, **caractérisé en ce qu'**on introduit l'effluent de tête de la première colonne de distillation dans la zone centrale de la deuxième colonne de distillation, de préférence au dixième jusqu'au vingtième plateau de la deuxième colonne de distillation.

5. Procédé suivant l'une des revendications 1 à 4, **caractérisé en ce que** la fraction du composant alcoolique respectif est de l'ordre de 10-90 % en poids, de préférence de 20-80 % en poids, et particulièrement avantageusement de 30-70 % en poids, par rapport au mélange d'alcools.

6. Procédé suivant l'une des revendications 1 à 5, **caractérisé en ce que** la réaction d'estérification est effectuée en présence d'un catalyseur d'estérification à une concentration de 0,5 à 3 % en poids, par rapport à la totalité du mélange réactionnel.

7. Procédé suivant l'une des revendications 1 à 6, **caractérisé en ce que** le réacteur d'estérification est une colonne d'estérification présentant un fond de colonne et une tour montée dessus.
